# EUROPEAN PATENT APPLICATION

(11) **EP 2 298 885 A1**
(43) Date of publication of application: **23.03.2011**
(21) Application number: 09754811.9
(22) Date of filing: 29.05.2009
(51) Int. Cl.: C12N 15/09, C12Q 1/68

(54) **PRIMER FOR DETECTION OF TYROSINASE mRNA**

(30) Priority: 29.05.2008 JP 2008141665
(71) Applicant: Sysmex Corporation, Kobe-shi, Hyogo 651-0073 (JP)
(72) Inventor: HIYAMA, Kayo, Kobe-shi Hyogo 651-0073 (JP); NAKABAYASHI, Kazuki, Kobe-shi Hyogo 651-0073 (JP); OTOMO, Yasuhiro, Kobe-shi Hyogo 651-0073 (JP)
(74) Representative: Paemen, Liesbet R.J.
(86) International application number: PCT/JP2009/059876
(87) International publication number: WO 2009/145303

(57) **Abstract**

The present invention provides a means and method for detecting TYR mRNA. As a primer for detecting TYP mRNA, a primer comprising a first sequence at the 5' end side and a second sequence at the 3' end side, wherein the first sequence has 10 to 30 nucleotides in length, and the first sequence is a sequence of a polynucleotide capable of hybridizing with a strand complementary to a first region which is a partial region of SEQ ID NO: 1; and wherein the second sequence has 10 to 30 nucleotides in length, and the second sequence is a sequence of a polynucleotide capable of hybridizing with a second region located in the 3' end side from the first region in SEQ ID NO^{:} 1 is used.

## Description

### TECHNICAL FIELD

The present invention relates to a primer used for detecting tyrosinase (hereinafter, referred to as "TYR") mRNA. More specifically, the present invention relates to a primer, a primer set and a method for detecting TYR mRNA, which are suitable for detection of TYR mRNA in a sample.

### BACKGROUND ART

TYR is expressed in an animal cell or plant cell and is one of enzymes catalyzing a reaction oxidizing tyrosine to synthesize melanin. According to studies in recent years, it has been revealed that TYR is useful as a molecular marker for detecting lymph node metastasis in melanoma. For example, Non-Patent Literature 1 discloses that the presence or absence of lymph node metastasis in melanoma can be determined by preparing a measurement sample from lymph node collected from a living body and detecting TYR mRNA in the measurement sample by RT-PCR.

### CITATION LIST

### NON-PATENT LITERATURE

Non-Patent Literature 1: Abrahamsen et al., "Quantification of Melanoma mRNA Markers in Sentinel Nodes: Pre-Clinical Evaluation of a Single-Step Real-Time ReverseTranscriptase-Polymerase Chain Reaction Assay," August 2004, Vol. 6, No. 3, p.253-259

### SUMMARY OF INVENTION

As described above, TYR mRNA is useful as a molecular marker for detecting cancer such as melanoma. However, since RT-PCR which can detect a target nucleic acid with high sensitivity is used upon detection in the technology described in Non-Patent Literature 1, for example, even microscopic metastasis to lymph node of cancer cell can be detected, it has been spent lots of time for the detection (2 to 4 hours or so).

An object of the present invention is to provide a primer for detecting TYR mRNA, a primer set for detecting TYR mRNA, a detection reagent kit for TYR mRNA and a method for detecting TYR mRNA which can detect TYR mRNA in a shorter time than the time required in the conventional technology.

That is, the present invention relates to:
[1] a primer for detecting tyrosinase mRNA used for detection of tyrosinase mRNA in a sample, comprising a first sequence at the 5' end side and a second sequence at the 3' end side,
   wherein the first sequence is defined by the following (a1) and (b1):
   (a1) having 10 to 30 nucleotides in length, and
   (b1) being a sequence of a polynucleotide capable of hybridizing with a strand complementary to a first region which is a partial region of SEQ ID NO: 1,
   the second sequence is defined by the following (c1) and (d1):
   (c1) having 10 to 30 nucleotides in length, and
   (d1) being a sequence of a polynucleotide capable of hybridizing with a second region located in the 3' end side from the first region in SEQ ID NO: 1, and
   the second region is a region comprising:
   (i) the 901st and 902nd nucleotides of SEQ ID NO: 1, or
   (ii) the 1118th and 1119th nucleotides of SEQ ID NO: 1,
[2] a primer set for detecting tyrosinase mRNA used for detection of tyrosinase mRNA in a sample, comprising a first primer, a second primer, and a third primer,
   wherein the first primer is the primer of in claim 1,
   the second primer comprises a third sequence at the 5' end side and a fourth sequence at the 3' end side,
   the third sequence is defined by the following (a2) and (b2):
   (a2) having 10 to 30 nucleotides in length, and
   (b2) being a sequence of a polynucleotide capable of hybridizing with a third region located in the 5' end side from the first region in SEQ ID NO: 1,
   the fourth sequence is defined by the following (c2) and (d2):
   (c2) having 10 to 30 nucleotides in length, and
   (d2) being a sequence of a polynucleotide capable of hybridizing with a strand complementary to a fourth region located in the 5' end side from the third region in SEQ ID NO: 1, and
   the third primer is a primer comprising a sequence of a polynucleotide capable of hybridizing with a strand complementary to a fifth region located in the 5' end side from the fourth region in SEQ ID NO: 1;
[3] a detection reagent kit for tyrosinase mRNA comprising:
   the primer set of the item [2],
   dNTPs, and
   an enzyme having an action of synthesizing DNA with RNA as a template and an action of synthesizing DNA with DNA as a template while performing strand displacement, or both of RNA-dependent DNA polymerase and DNA-dependent DNA polymerase;
[4] a method for detecting tyrosinase mRNA, comprising the steps of:
   reacting a sample, the primer set of the item [2], and RNA-dependent DNA polymerase, to synthesize cDNA from tyrosinase mRNA in the sample,
   reacting the primer set, DNA-dependent DNA polymerase, and the cDNA synthesized in the above step, to amplify the cDNA, and
   detecting the cDNA amplified in the above step, thereby detecting tyrosinase mRNA in the sample; and
[5] a method for detecting tyrosinase mRNA, comprising the steps of:
   reacting a sample, the primer set of the item [2], and an enzyme having an action of synthesizing DNA with RNA as a template and an action of synthesizing DNA with DNA as a template while performing strand displacement, to synthesize cDNA with tyrosinase mRNA in the sample as a template and to additionally synthesize and amplify the cDNA with the cDNA as a template, and
   detecting the cDNA amplified in the above step, thereby detecting tyrosinase mRNA in the sample.

According to the primer for detecting TYR mRNA, the primer set for detecting TYR mRNA, the detection reagent kit for TYR mRNA and the method for detecting TYR mRNA of the present invention, TYR mRNA can be detected in a short time.

### BRIEF DESCRIPTION OF DRAWINGS

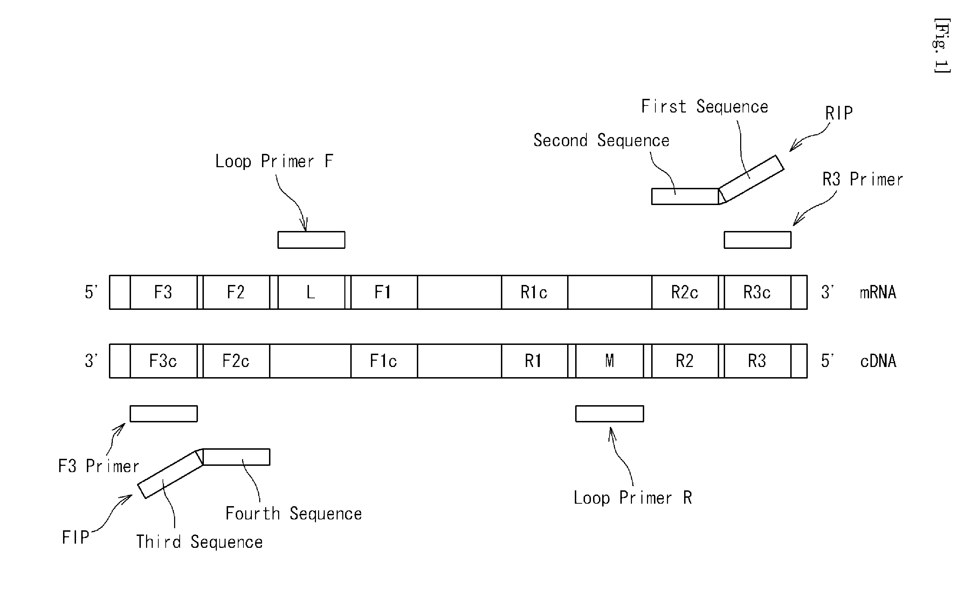

Fig. 1 is a schematic view showing a primer and a region with which the primer hybridizes.

### MODE FOR CARRING OUT THE INVENTION

### [Primer and Primer Set]

First, a primer for detecting TYR mRNA used for detection of TYR mRNA will be described.

Incidentally, the term "detecting" in the specification encompasses not only determination of whether or not TYR mRNA which is a target substance is present in a sample, but also quantification of TYR mRNA in a sample.

The term "primer" in the specification refers to a polynucleotide having a function capable of hybridizing with a specific region of a nucleic acid to be amplified to become the start point of the amplification reaction by polymerase (hereinafter, referred to as "primer function") in a nucleic acid amplification technique such as LAMP (Loop-mediated Isothermal Amplification) method (see, US Patent No. 6,410,278 and US Patent No. 6,974,670). Since the nucleic acid to be detected is mRNA, it can be detected by a nucleic acid amplification reaction including a reverse transcription reaction (for example, RT LAMP method and the like).

The term "hybridize" means that a part or all of a polynucleotide binds to a part or all of another polynucleotide through hydrogen bonding based on the complementarities of bases in a polynucleotide under stringent conditions.

The term "stringent condition" refers to the condition generally used by those skilled in the art when hybridization between polynucleotides is carried out, and is not particularly limited as long as it is a condition in which the primer of the embodiment can hybridize with TYR mRNA or cDNA thereof. The stringency in hybridization is known to be functions of the temperature, the salt concentration, the chain length of a primer, the GC content of a primer, and the concentration of a chaotropic agent in a hybridization buffer solution. As the stringent conditions, for example, the conditions described in Sambrook, J. et al., 1998, Molecular Cloning: A Laboratory Manual (2nd ed.), Cold Spring Harbor Laboratory Press, New York, and the like can be used. More specifically, "hybridization temperature of 42°C in a solution containing 50% formamide, 5 x SSC (150 mM NaCl, 15 mM sodium citrate), 50 mM sodium phosphate, pH 7.6, 5 x Denhardt's solution, 10% dextran sulfuric acid, and 20 µg/ml of nucleic acid" can be exemplified, but is not limited thereto.

A nucleotide sequence of TYR mRNA is a sequence corresponding to a sequence as shown in SEQ ID NO: 1. Incidentally, while mRNA does not contain thymine and contains uracil in place of thymine, uracil is represented as thymine (t) in SEQ ID NO: 1 for convenience. This sequence is registered as Accession No. NM_000372 in GenBank database.

The primer according to one embodiment (Embodiment 1) of the present invention is a primer for detecting TYR mRNA used for detection of tyrosinase mRNA in a sample, comprising a first sequence at the 5' end side and a second sequence at the 3' end side,
wherein the first sequence is defined by the following (a1) and (b1):
(a1) having 10 to 30 nucleotides in length, and
(b1) being a sequence of a polynucleotide capable of hybridizing with a strand complementary to a first region which is a partial region of SEQ ID NO: 1,
the second sequence is defined by the following (c1) and (b1):
(c1) having 10 to 30 nucleotides in length, and
(d1) being a sequence of a polynucleotide capable of hybridizing with a second region located in the 3' end side from the first region in SEQ ID NO: 1, and
the second region is a region comprising:
(i) the 901st and 902nd nucleotides of SEQ ID NO: 1; or
(ii) the 1118th and 1119th nucleotides of SEQ ID NO: 1.

The primer of Embodiment 1 is a primer suitably used for, especially, the detection of TYR mRNA using RT-LAMP method. According to the primer of Embodiment 1, since the primer comprises the first sequence at the 5' end side and the second sequence at the 3' end side, TYR mRNA can be efficiently and reproducibly detected.

In the RT-LAMP method, cDNA is first synthesized from TYR mRNA by reverse transcription reaction (RT reaction), and subsequently the synthesized cDNA is amplified by LAMP reaction.
In the detection of TYR mRNA using this RT-LAMP method, there can be used as the primer of Embodiment 1, for example, a primer comprising a polynucleotide having a first sequence which is a sequence of a polynucleotide capable of hybridizing with a complementary region ("R1" in Fig. 1) of a partial region of TYR mRNA ("R1c" in Fig. 1) at the 5' end side and having a second sequence which is a sequence of a polynucleotide capable of hybridizing with a region located in the downstream (3' end side) from the region R1c in TYR mRNA ("R2c" in Fig. 1), as schematically shown in Fig. 1.

The first sequence and the second sequence each dependently have preferably 10 nucleotides or more in length from the viewpoint of specificity of the hybridization between the primer of Embodiment 1 and a region with which the primer hybridizes and have preferably 30 nucleotides or less in length from the viewpoint of securing the hybridization temperature easy to operate in the detection of TYR mRNA.

In the primer of Embodiment 1, the first sequence and the second sequence may be directly ligated. The sequences may be ligated through an intervening sequence. The intervening sequence is preferably a sequence having low relevancy with TYR mRNA and TYR cDNA. For example, 5'-tttt-3' and the like can be exemplified. The length of the intervening sequence is preferably 1 to 50 nucleotides, and more preferably 1 to 40 nucleotides.

The primer of Embodiment 1 can function as a reverse inner primer (hereinafter referred to as "RIP") among the primers used in the RT-LAMP method.

In order to detect TYR mRNA using the RT-LAMP method, the primer of Embodiment 1 (RIP), a forward inner primer (hereinafter referred to as "FIP"), and F3 primer can be used. The present invention also encompasses a primer set for detecting TYR mRNA comprising these primers.

The primer set for detecting TYR mRNA according to one embodiment of the present invention is a primer set for detecting TYR mRNA used for detection of tyrosinase mRNA in a sample, comprising a first primer, a second primer, and a third primer,
wherein the first primer is the primer of Embodiment 1,
the second primer comprises a third sequence at the 5' end side and a fourth sequence at the 3' end side,
the third sequence is defined by the following (a2) and (b2):
(a2) having 10 to 30 nucleotides in length, and
(b2) being a sequence of a polynucleotide capable of hybridizing with a third region located in the 5' end side from the first region in SEQ ID NO: 1,
the fourth sequence is defined by the following (c2) and (d2):
(c2) having 10 to 30 nucleotides in length, and
(d2) being a sequence of a polynucleotide capable of hybridizing with a strand complementary to a fourth region located in the 5' end side from the third region in SEQ ID NO: 1, and
the third primer is a primer comprising a sequence of a polynucleotide capable of hybridizing with a strand complementary to a fifth region located in the 5' end side from the fourth region in SEQ ID NO: 1.

According to the primer set of the embodiment, since the primer set comprises the first primer, the second primer and the third primer, TYR mRNA can be rapidly and reproducibly detected with high efficiency.

The primer set of the embodiment may further comprise a fourth primer capable of hybridizing with a sixth region located in the 3' end side from the second region in SEQ ID NO: 1. In addition, the primer set of the embodiment may further comprise a fifth primer which hybridizes with a seventh region located between the third region and the fourth region in SEQ ID NO: 1. Furthermore, the primer set of the embodiment may further comprise a sixth primer that hybridizes with a region complementary to an eighth region located between the first region and the second region in SEQ ID NO: 1.

Hereinafter, the above primer and primer set will be further described based on Fig. 1.
Fig. 1 is a schematic view showing a primer and a region with which the primer hybridizes. Incidentally, each of the regions of F1, F2, L, F1, R1c, R2c and R3c in Fig. 1 is a region on TYR mRNA. Each of the regions of F3c, F2c, F1c, R1, M, R2 and R3 is a region on TYR cDNA which is a strand complementary to TYR mRNA. In addition, each of F1 and F1c, each of F2 and F2c, each of F3 and F3c, each of R1 and R1c, each of R2 and R2c, and each of R3 and R3c are complementary each other. These regions are selected in consideration of the detection efficiency and reproducibility of TYR mRNA, and the like.

In Fig. 1, RIP corresponds to the first primer, FIP being the second primer, F3 primer being the third primer, R3 primer being the fourth primer, loop primer F being the fifth primer and loop primer R being the sixth primer.

FIP (the second primer) is a polynucleotide having at the 5' end side the third sequence which is a sequence of a polynucleotide capable of hybridizing with the region F1 (region complementary to F1c) of TYR cDNA which is a strand complementary to TYR mRNA and having at the 3' end side the fourth sequence which is a sequence of a polynucleotide capable of hybridizing with the region F2c of TYR cDNA. The third sequence and the fourth sequence may be directly ligated. The sequences may be ligated through the intervening sequence. In addition, in FIP, the lengths of the third sequence and the fourth sequence are the same as the lengths of the first sequence and the second sequence.

F3 primer (third primer) comprises a polynucleotide capable of hybridizing with the region F3c located in the downstream (3' end side) from F2c of TYR mRNA.

Furthermore, the primer set of the present invention may comprise R3 primer in addition to FIP, RIP and F3 primer. This R3 primer comprises a polynucleotide capable of hybridizing with the region R3c located in the downstream (3' end side) from R2c of TYR mRNA.

Furthermore, the primer set of the present invention may comprise a loop primer. The loop primer includes loop primer F which is a polynucleotide capable of hybridizing with the region L located between F2 and F1 in TYR mRNA, loop primer R which is a polynucleotide capable of hybridizing with the region M located between R1 and R2 in TYR cDNA, and the like. The primer set of the present invention may comprise one or both of these loop primers F and R.
When the primer set of the present invention comprises one or both of loop primers F and R, amplification of cDNA by LAMP reaction can be further rapidly carried out according to this primer set.

The chain lengths of F3 primer, R3 primer and loop primers F and R are not particularly limited as long as it is a length enough to exhibit primer function. Since the chain length of the primer which is recognized by known polymerase catalyzing a nucleic acid synthesis reaction is 5 nucleotides or more, the chain length of each of the primers is preferably 5 to 100 nucleotides. In addition, considering the specificity between a nucleotide sequence of the primer and a region with which the primer hybridizes, the chain length of each of the primers is preferably 10 nucleotides or more. Also, considering the hybridization temperature, the chain length of each of the primers is preferably 30 nucleotides or less. In addition, the chain lengths of FIP and RIP are preferably 20 to 200 nucleotides and more preferably 20 to 60 nucleotides.

In the primer set of the present invention, it is preferred that at least one primer among the primers described above hybridizes with a region comprising an exon junction of TYR mRNA. TYR gene comprises 9 exons, and an intron intervenes between exon and exon. When mRNA is synthesized from the TYR gene in a cell, the intron is removed by splicing, so that the exons are directly ligated. A portion of this junction where exon and exon are ligated is referred to as exon junction. For example, exon 1 and exon 2 are separated by an intron in a genome. However, the intron is removed in mRNA synthesis, the 3' end of exon 1 is adjacent to the 5' end of exon 2 in mRNA. Since SEQ ID NO: 1 comprises 5 exons, SEQ ID NO: 1 contains the following 4 exon junctions (exon junctions 1 to 4).
Exon junction 1 of exons 1 and 2: Junction of the 901st and the 902nd in SEQ ID NO: 1
Exon junction 2 of exons 2 and 3: Junction of the 1118th and the 1119th in SEQ ID NO: 1
Exon junction 3 of exons 3 and 4: Junction of the 1266th and the 1267th in SEQ ID NO: 1
Exon junction 4 of exons 4 and 5: Junction of the 1448th and the 1449th in SEQ ID NO: 1

A primer capable of hybridizing with a region comprising an exon junction hybridizes with mRNA of TYR gene. However, the primer is very unlikely to hybridize with genomic DNA of TYR gene. According to such primer as described above, nonspecific amplification of nucleic acid can be prevented upon mRNA detection by RT-LAMP reaction.

In addition, according to the reaction mechanism of RT-LAMP reaction (see US Patent No. 6,410,278 and US Patent No. 6,974,670), one that initially hybridizes with TYR mRNA in a sample is considered to be a polynucleotide portion comprising a second sequence of RIP. Therefore, in the primer set of the present invention, it is preferred that the polynucleotide portion comprising the second sequence of RIP hybridizes with a region comprising an exon junction among the primers described above. In other words, it is more preferred that the region R2c comprises an exon junction. Accordingly nonspecific amplification of nucleic acid can be provided in the early stage of the reaction.

From the viewpoint of the homology between TYR mRNA and mRNA derived from other genes in a sample, detection rate, reproducibility of detection, and the like, the primer set of the present invention comprises preferably a primer hybridizing with a region comprising exon junction 1 or 2, and more preferably a primer hybridizing with a region comprising exon junction 2 of the exon junctions described above.

The primer of the embodiment is not required to be completely mutually complementary to a specific region of TYR mRNA with which this primer hybridizes. Both primers may have complementarity to an extent that both can mutually hybridize (also described in US Patent No. 4,800,159). In other words, this primer may be a polynucleotide having one or plural, i.e., several mutations such as substitution, deletion, insertion, addition and the like in a sequence having complete complementarity to the specific region as long as the primer is a polynucleotide having a primer function. The polynucleotide having this mutation has preferably 80% or more homology, more preferably 90% or more homology, and further more preferably 95% or more homology to a polynucleotide having no mutation.

In RT-LAMP reaction, after a primer hybridizes with TYR mRNA which is a target nucleic acid, nucleic acid synthesis is carried out using the 3' end of the primer as the start point by the action of polymerase. Thus, it is considered that, the higher the complementarity between the 3' end portion of the primer and TYR mRNA, the easier a nucleic acid synthesis reaction proceeds. Therefore, the primer of the present invention is a polynucleotide in which preferably 3 nucleotides at the 3' end is completely complementary and more preferably 5 nucleotides at the 3' end is completely complementary to a region with which the primer hybridizes.

Among them, from the viewpoint of improving specificity in the detection of TYR mRNA, the second sequence preferably comprises at the 3' end a sequence consisting of 3 consecutive nucleotides and being completely complementary to a sequence of the second region, and more preferably comprises at the 3' end a sequence consisting of 5 consecutive nucleotides and being completely complementary to a sequence of the second region.

Each of the regions of F3, F2, F1, R1c, R2c, R3c, L and M is arranged in the sequence of TYR mRNA as shown in SEQ ID NO: 1. Concrete examples of these regions are listed hereinbelow.

(F1)
a region from the 795th to 817th
a region from the 1005th to 1024th
a region from the 1015th to 1036th
a region from the 1016th to 1040th
a region from the 1031st to 1054th

(F2)
a region from the 731st to 751st
a region from the 963rd to 977th
a region from the 964th to 986th
a region from the 971st to 992nd
a region from the 972nd to 992nd
a region from the 986th to 1008th

(F3)
a region from the 703rd to 722nd
a region from the 931st to 953rd
a region from the 945th to 962nd a region from the 950th to 962nd a region from the 966th to 985th

(R1c)
a region from the 825th to 848th
a region from the 1026th to 1049th
a region from the 1041st to 1062nd
a region from the 1044th to 1064th

a region from the 1044th to 1068th a region from the 1057th to 1080th

(R2c)
a region from the 886th to 908th
a region from the 1110th to 1133rd
a region from the 1114th to 1131st
a region from the 1114th to 1133rd

(R3c)
a region from the 917th to 936th
a region from the 1135th to 1153rd
a region from the 1138th to 1155th
a region from the 1138th to 1156th
a region from the 1141st to 1160th
a region from the 1142nd to 1161st

(L)
a region from the 752nd to 776th
a region from the 981st to 1004th
a region from the 982nd to 1004th
a region from the 982nd to 1006th
a region from the 986th to 1010th
a region from the 987th to 1011st
a region from the 988th to 1011st
a region from the 988th to 1012nd
a region from the 989th to 1011st
a region from the 989th to 1012nd
a region from the 989th to 1013rd
a region from the 990th to 1012nd
a region from the 990th to 1013rd
a region from the 990th to 1014th
a region from the 992nd to 1015th
a region from the 993rd to 1016th
a region from the 994th to 1016th
a region from the 995th to 1016th
a region from the 995th to 1018th
a region from the 996th to 1018th
a region from the 1000th to 1019th
a region from the 1001st to 1020th
a region from the 1003rd to 1022nd
a region from the 1004th to 1022nd
a region from the 1005th to 1024th
a region from the 1006th to 1024th
a region from the 1008th to 1027th
a region from the 1009th to 1027th
a region from the 1009th to 1028th
a region from the 1010th to 1029th

(M)
a region from the 860th to 884th
a region from the 1063rd to 1087th
a region from the 1065th to 1089th
a region from the 1066th to 1090th
a region from the 1067th to 1089th
a region from the 1067th to 1090th
a region from the 1068th to 1091st
a region from the 1068th to 1092nd
a region from the 1069th to 1091st
a region from the 1069th to 1092nd
a region from the 1069th to 1093rd
a region from the 1070th to 1093rd
a region from the 1070th to 1094th
a region from the 1073rd to 1097th
a region from the 1074th to 1098th
a region from the 1075th to 1099th
a region from the 1082nd to 1104th
a region from the 1083rd to 1105th
a region from the 1084th to 1105th
a region from the 1085th to 1107th

In the present invention, the primer can be designed based on each region described above. Concrete examples of each primer are listed hereinbelow. Incidentally, the description in parentheses after the sequence shows that each primer is the same sequence as or complementary to a give region of the sequence of TYR, mRNA (SEQ ID NO: 1).

(F3 Primer)
SEQ ID NO: 2: 5'-AATGGAACGCCCGAGGGA-3' (the same sequence as a region from the 950th to 967th)
SEQ ID NO: 3: 5'-GACCTTTACGGCGTAATCCT-3' (the same sequence as a region from the 966th to 985th)
SEQ ID NO: 4: 5'-TATGCAATGGAACGCCCG-3' (the same sequence as a region from the 945th to 962nd)
SEQ ID NO: 5: 5'-CAGCCATCAGTCTTTATGCAATG-3' (the same sequence as a region from the 931st to 953rd)
SEQ ID NO: 6: 5'-TCTGCCTTGGCATAGACTCT-3' (the same sequence as a region from the 703rd to 722nd)

(FIP)
SEQ ID NO: 7: 5'-CAAACTCAGGCAAAATTCTACATCTTACGGCGTAATCCTGGAAACC-3'
(a sequence obtained by ligating a sequence complementary to a region from the 1031st to 1054th and the same sequence as a region from the 971st to 992nd)
SEQ ID NO: 8: 5'-CAAACTCAGGCAAAATTCTACATCGGAAACCATGACAAATCCAGAAC-3' (a sequence obtained by ligating a sequence complementary to a region from the 1031st to 1054th and the same sequence as a region from the 986th to 1008th)
SEQ ID NO: 9: 5'-TACATCAGCTGAAGAGGGGAGCAGGGACCTTTACGGC-3' (a sequence obtained by ligating a sequence complementary to a region from the 1015th to 1036th and the same sequence as a region from the 963rd to 977th)
SEQ ID NO: 10: 5'-CAAACTCAGGCAAAATTCTACATCTACGGCGTAATCCTGGAAACC-3'
(a sequence obtained by ligating a sequence complementary to a region from the 1031st to 1054th and the same sequence as a region from the 972nd to 992nd)
SEQ ID NO: 11: 5'-AGAGGGGAGCCTTGGGGTTCAGGGACCTTTACGGC-3' (a sequence obtained by ligating a sequence complementary to a region from the 1005th to 1024th and the same sequence as a region from the 963rd to 977th)
SEQ ID NO: 12: 5'-ATTCTACATCAGCTGAAGAGGGGAGGGGACCTTTACGGCGTAATCCT G-3' (a sequence obtained by ligating a sequence complementary to a region from the 1016th to 1040th and the same sequence as a region from the 964th to 986th)
SEQ ID NO: 13: 5'-GTCACACTTTTCTGCATCCCGCCCGGTGGGAACAAGAAATCCAG-3'
(a sequence obtained by ligating a sequence complementary to a region from the 795th to 817th and the same sequence as a region from the 731st to 751st)

(RIP)
SEQ ID NO: 14: 5'-CCAATATGAATCTGGTTCCATGGAGTGGACTAGCAAATCCTTCC-3'
(a sequence obtained by ligating the same sequence as a region of the 1057th to 1080th and a sequence complementary to a region from the 1114th to 1133rd)
SEQ ID NO: 15: 5'-TTTGCCTGAGTTTGACCCAATAGGACTAGCAAATCCTTCC-3' (a
sequence obtained by ligating the same sequence as a region of the 1041st to 1062nd and a sequence complementary to a region from the 1114th to 1131st)
SEQ ID NO: 16: 5'-GCCTGAGTTTGACCCAATATGGTGGACTAGCAAATCCTTCC-3' (a sequence obtained by ligating the same sequence as a region of the 1044th to 1064th and a sequence complementary to a region from the 1114th to 1133rd)
SEQ ID NO: 17: 5'-CAGCTGATGTAGAATTTTGCCTGAGTGGACTAGCAAATCCTTCC-3'
(a sequence obtained by ligating the same sequence as a region of the 1026th to 1049th and a sequence complementary to a region from the 1114th to 1133rd)
SEQ ID NO: 18: 5'-GCCTGAGTTTGACCCAATATGAATCGTGGACTAGCAAATCCTTCCAGT G-3' (a sequence obtained by ligating the same sequence as a region of the 1044th to 1068th and a sequence complementary to a region from the 1110th to 1133rd)
SEQ ID NO: 19: 5'-CAGATGAGTACATGGGAGGTCAGCAGACAATCTGCCAAGAGGAGAAG -3' (a sequence obtained by ligating the same sequence as a region of the 825th to 848th and a sequence complementary to a region from the 886th to 908th)

(R3 Primer)
SEQ ID NO: 20: 5'-GAGGCATCCGCTATCCCA-3' (a sequence complementary to a region from the 1138th to 1155th)
SEQ ID NO: 21: 5'-TTTGAGAGGCATCCGCTATC-3' (a sequence complementary to a region from the 1141st to 1160th)
SEQ ID NO: 22: 5'-GGCATCCGCTATCCCAGTA-3' (a sequence complementary to a region from the 1135th to 1153rd)
SEQ ID NO: 23: 5'-AGAGGCATCCGCTATCCCA-3' (a sequence complementary to a region from the 1138th to 1156th)
SEQ ID NO: 24: 5'-CTTTGAGAGGCATCCGCTAT-3' (a sequence complementary to a region from the 1142nd to 1161st)
SEQ ID NO: 25: 5'-TGGCTGTTGTACTCCTCCAA-3' (a sequence complementary to a region from the 917th to 936th)

(Loop Primer F)
SEQ ID NO: 26: 5'-GCCTTGGGGTTCTGGATTTGTCA-3' (a sequence complementary to a region from the 994th to 1016th)
SEQ ID NO: 27: 5'-CTGAAGAGGGGAGCCTTGGG-3' (a sequence complementary to a region from the 1009th to 1028th)
SEQ ID NO: 28: 5'-GCCTTGGGGTTCTGGATTTGTCAT-3' (a sequence complementary to a region from the 993rd to 1016th)
SEQ ID NO: 29: 5'-TGAAGAGGGGAGCCTTGGG-3'(a sequence complementary to a region from the 1009th to 1027th)
SEQ ID NO: 30: 5'-GGAGCCTTGGGGTTCTGGAT-3' (a sequence complementary to a region from the 1000th to 1019th)
SEQ ID NO: 31: 5'-GCCTTGGGGTTCTGGATTTGTC-3' (a sequence complementary to a region from the 995th to 1016th)
SEQ ID NO: 32: 5'-GGGTTCTGGATTTGTCATGGTTTCC-3' (a sequence complementary to a region from the 986th to 1010th)
SEQ ID NO: 33: 5'-GGGAGCCTTGGGGTTCTGGA-3' (a sequence complementary to a region from the 1001st to 1020th)
SEQ ID NO: 34: 5'-GAGCCTTGGGGTTCTGGATTTGT-3' (a sequence complementary to a region from the 996th to 1018th)
SEQ ID NO: 35: 5'-GCTGAAGAGGGGAGCCTTGG-3' (a sequence complementary to a region from the 1010th to 1029th)
SEQ ID NO: 36: 5'-TCTGGATTTGTCATGGTTTCCAGGA-3' (a sequence complementary to a region from the 982nd to 1006th)
SEQ ID NO: 37: 5'-GAGCCTTGGGGTTCTGGATTTGTC-3' (a
sequence complementary to a region from the 995th to 1018th)
SEQ ID NO: 38: 5'-TGGGGTTCTGGATTTGTCATGGTT-3' (a sequence complementary to a region from the 989th to 1012nd)
SEQ ID NO: 39: 5'-GGGGTTCTGGATTTGTCATGGTTTC-3' (a sequence complementary to a region from the 987th to 1011st)
SEQ ID NO: 40: 5'-TGAAGAGGGGAGCCTTGGGG-3' (a sequence complementary to a region from the 1008th to 1027th)
SEQ ID NO: 41: 5'-CTTGGGGTTCTGGATTTGTCATGGT-3' (a sequence complementary to a region from the 990th to 1014th)
SEQ ID NO: 42: 5'-TGGGGTTCTGGATTTGTCATGGT-3' (a sequence complementary to a region from the 990th to 1012nd)
SEQ ID NO: 43: 5'-AGGGGAGCCTTGGGGTTCT-3' (a sequence complementary to a region from the 1004th to 1022nd)
SEQ ID NO: 44: 5'-AGAGGGGAGCCTTGGGGTTC-3' (a sequence complementary to a region from the 1005th to 1024th)
SEQ ID NO: 45: 5'-TTGGGGTTCTGGATTTGTCATGGT-3' (a sequence complementary to a region from the 990th to 1013rd)
SEQ ID NO: 46: 5'-AGAGGGGAGCCTTGGGGTT-3' (a sequence complementary to a region from the 1006th to 1024th)
SEQ ID NO: 47: 5'-TGGATTTGTCATGGTTTCCAGGAT-3' (a sequence complementary to a region from the 981st to 1004th)
SEQ ID NO: 48: 5'-TGGGGTTCTGGATTTGTCATGGTTT-3' (a
sequence complementary to a region from the 988th to 1012nd)
SEQ ID NO: 49: 5'-GGGGTTCTGGATTTGTCATGGTT-3' (a sequence complementary to a region from the 989th to 1011st)
SEQ ID NO: 50: 5'-CCTTGGGGTTCTGGATTTGTCATG-3' (a sequence complementary to a region from the 992nd to 1015th)
SEQ ID NO: 51: 5'-TGGATTTGTCATGGTTTCCAGGA-3' (a sequence complementary to a region from the 982nd to 1004th)
SEQ ID NO: 52: 5'-GGGGTTCTGGATTTGTCATGGTTT-3' (a sequence complementary to a region from the 988th to 1011st)
SEQ ID NO: 53: 5'-TTGGGGTTCTGGATTTGTCATGGTT-3' (a sequence complementary to a region from the 989th to 1013rd)
SEQ ID NO: 54: 5'-AGGGGAGCCTTGGGGTTCTG-3' (a sequence complementary to a region from the 1003rd to 1022nd)
SEQ ID NO: 55: 5'-TGAAGTTTTCATCTCCTGTCAGCTT-3' (a sequence complementary to a region from the 752nd to 776th)

(Loop Primer R)
SEQ ID NO: 56: 5'-AAAGCTGCCAATTTCAGCTTTAG-3' (the same sequence as a region of the 1082nd to 1104th)
SEQ ID NO: 57: 5'-AGCTGCCAATTTCAGCTTTAGA-3' (the same sequence as a region of the 1084th to 1105th)
SEQ ID NO: 58: 5'-GCTGCCAATTTCAGCTTTAGAAA-3' (the same sequence as a region of the 1085th to 1107th)
SEQ ID NO: 59: 5'-AAGCTGCCAATTTCAGCTTTAGA-3' (the same sequence as a region of the 1083rd to 1105th)
SEQ ID NO: 60: 5'-ATCTGGTTCCATGGATAAAGCTGCC-3' (the same sequence as a region of the 1066th to 1090th)
SEQ ID NO: 61: 5'-AATCTGGTTCCATGGATAAAGCTGC-3' (the same sequence as a region of the 1065th to 1089th)
SEQ ID NO: 62: 5'-CTGGTTCCATGGATAAAGCTGCCAA-3' (the same sequence as a region of the 1068th to 1092nd)
SEQ ID NO: 63: 5'-TGGTTCCATGGATAAAGCTGCCAA-3' (the same sequence as a region of the 1069th to 1092nd)
SEQ ID NO: 64: 5'-GGTTCCATGGATAAAGCTGCCAAT-3' (the same sequence as a region of the 1070th to 1093rd)
SEQ ID NO: 65: 5'-TCCATGGATAAAGCTGCCAATTTCA-3' (the same sequence as a region of the 1073rd to 1097th)
SEQ ID NO: 66: 5'-CTGGTTCCATGGATAAAGCTGCCA-3' (the same sequence as a region of the 1068th to 1091st)
SEQ ID NO: 67: 5'-TCTGGTTCCATGGATAAAGCTGCC-3' (the same sequence as a region of the 1067th to 1090th)
SEQ ID NO: 68: 5'-TGAATCTGGTTCCATGGATAAAGCT-3' (the same sequence as a region of the 1063rd to 1087th)
SEQ ID NO: 69: 5'-TGGTTCCATGGATAAAGCTGCCA-3' (the same sequence as a region of the 1069th to 1091st)
SEQ ID NO: 70: 5'-CATGGATAAAGCTGCCAATTTCAGC-3' (the same sequence as a region of the 1075th to 1099th)
SEQ ID NO: 71: 5'-GGTTCCATGGATAAAGCTGCCAATT-3' (the same sequence as a region of the 1070th to 1094th)
SEQ ID NO: 72: 5'-CCATGGATAAAGCTGCCAATTTCAG -3'(the same sequence as a region of the 1074th to 1098th)
SEQ ID NO: 73: 5'-TCTGGTTCCATGGATAAAGCTGC-3' (the same sequence as a region of the 1067th to 1089th)
SEQ ID NO: 74: 5'-TGGTTCCATGGATAAAGCTGCCAAT-3' (the same sequence as a region of the 1069th to 1093rd)
SEQ ID NO: 75: 5'-CCTAACTTACTCAGCCCAGCATCAT-3' (the same sequence as a region of the 860th to 884th)

Among the primers listed above, the primer comprising a nucleotide sequence as shown in SEQ ID NO: 19 is capable of hybridizing with a region comprising exon junction 1.
The primers of SEQ ID NOs: 14 to 18 are capable of hybridizing with a region comprising exon junction 2.

The above primers can be used as a primer set comprising 4 kinds of primers by properly combining F3 primer, FIP, RIP and R3 primer depending on an amplified region. In addition, the above primers can be used as a primer set comprising 6 kinds of primers by further combining loop primers F and R. Concrete examples of these primer sets are shown in the following Table 1.

**[Table 1]**

| Primer Set | F3 Primer | FIP | RIP | R3 Primer | Loop PrimerF | Loop PrimerR | | Primer Set | F3 Primer | FIP | RIP | R3 Primer | Loop PrimerF | Loop PrimerR |
|---|---|---|---|---|---|---|---|---|---|---|---|---|---|---|
| 1 | 2 | 7 | 14 | 20 | 26 | 56 | | 24 | 2 | 10 | 14 | 23 | 43 | 57 |
| 2 | 3 | 8 | 14 | 20 | 27 | 57 | | 25 | 2 | 10 | 14 | 23 | 44 | 57 |
| 3 | 2 | 7 | 14 | 20 | 28 | 58 | | 26 | 4 | 9 | 15 | 21 | 45 | 67 |
| 4 | 3 | 8 | 14 | 20 | 29 | 57 | | 27 | 2 | 10 | 14 | 23 | 46 | 57 |
| 5 | 2 | 7 | 14 | 20 | 30 | 59 | | 28 | 4 | 11 | 17 | 24 | 47 | 68 |
| 6 | 2 | 7 | 14 | 20 | 31 | 57 | | 29 | 5 | 12 | 18 | 22 | 48 | 63 |
| 7 | 4 | 9 | 15 | 21 | 32 | 60 | | 30 | 5 | 12 | 18 | 22 | 38 | 63 |
| 8 | 4 | 9 | 16 | 22 | 32 | 61 | | 31 | 5 | 12 | 18 | 22 | 39 | 69 |
| 9 | 2 | 7 | 14 | 20 | 33 | 57 | | 32 | 4 | 11 | 17 | 24 | 47 | 70 |
| 10 | 2 | 10 | 14 | 23 | 27 | 57 | | 33 | 4 | 11 | 17 | 24 | 47 | 64 |
| 11 | 2 | 7 | 14 | 20 | 34 | 57 | | 34 | 5 | 12 | 18 | 22 | 48 | 65 |
| 12 | 2 | 10 | 14 | 23 | 35 | 57 | | 35 | 5 | 12 | 18 | 22 | 49 | 63 |
| 13 | 4 | 9 | 16 | 22 | 36 | 60 | | 36 | 5 | 12 | 18 | 22 | 50 | 71 |
| 14 | 4 | 9 | 16 | 22 | 36 | 62 | | 37 | 4 | 11 | 17 | 24 | 51 | 72 |
| 15 | 2 | 7 | 14 | 20 | 37 | 58 | | 38 | 5 | 12 | 18 | 22 | 39 | 65 |
| 16 | 4 | 9 | 15 | 21 | 38 | 63 | | 39 | 4 | 11 | 17 | 24 | 51 | 61 |
| 17 | 4 | 9 | 16 | 22 | 39 | 64 | | 40 | 5 | 12 | 18 | 22 | 52 | 69 |
| 18 | 4 | 9 | 15 | 21 | 39 | 60 | | 41 | 4 | 11 | 17 | 24 | 51 | 73 |
| 19 | 4 | 9 | 16 | 22 | 32 | 65 | | 42 | 5 | 12 | 18 | 22 | 53 | 74 |
| 20 | 4 | 9 | 16 | 22 | 39 | 60 | | 43 | 5 | 12 | 18 | 22 | 50 | 65 |
| 21 | 2 | 10 | 14 | 23 | 40 | 57 | | 44 | 2 | 10 | 14 | 23 | 54 | 57 |
| 22 | 4 | 9 | 15 | 21 | 41 | 66 | | 45 | 6 | 13 | 19 | 25 | 55 | 75 |
| 23 | 4 | 9 | 15 | 21 | 42 | 60 | | | | | | | | |

As a reverse transcriptase and a strand displacement DNA polymerase, known enzymes can be used. In addition, in place of these tw enzymes (reverse transcriptase and strand displacement DNA polymerase), one kind of enzyme having an action of synthesizing DNA with RNA as a template and an action of synthesizing DNA as a template while performing strand displacement pay be used.

Furthermore, it is preferred that a buffer providing suitable conditions to an enzyme reaction is also used.
While the substances described above can be each stored in a separate container, the reverse transcriptase and the strand displacement DNA polymerase may be stored in the same container. In addition, at least two kinds of substances out of dNTPs, buffers, and various primers may be stored in the same container. When these reagents are provided to a user, these may be provided as a reagent kit comprising a part or all of the reagents described above.

### [Method for Detecting TYR mRNA and Detection Reagent Kit for TYR mRNA]

Next, a method for detecting TYR mRNA will be described. The method for detecting TYR mRNA of the present invention can be carried out by:
using the primer set described above and RNA-dependent DNA polymerase and DNA-dependent DNA polymerase ("detection method of Embodiment 1" as follows), or
using the primer set described above and an enzyme having an action of synthesizing DNA with RNA as a template and an action of synthesizing DNA with DNA as a template while performing strand displacement ("detection method of Embodiment 2" as follows).

The detection method of Embodiment 1 is a method comprising the steps of:
(I-1) reacting a sample with the primer set described above and RNA-dependent DNA polymerase, to synthesize cDNA from tyrosinase mRNA in the sample;
(II-1) reacting the cDNA synthesized in the above step (I-1) with the primer set and DNA-dependent DNA polymerase, to amplify the cDNA; and
(III-1) detecting the cDNA amplified in the above step (II-1), thereby detecting tyrosinase mRNA in the sample.

In addition, the detection method of Embodiment 2 is a method comprising the steps of:
(I-2) reacting a sample with the primer set described above and an enzyme having an action of synthesizing DNA with RNA as a template and an action of synthesizing DNA with DNA as a template while performing strand displacement, to synthesize cDNA with tyrosinase mRNA in the sample as a template and to addiltionally synthesize and amplify the cDNA with the cDNA as a template; and
(II-2) detecting the cDNA amplified in the above step, thereby detecting tyrosinase mRNA in the sample.

The detection methods of Embodiments 1 and 2 are preferably carried out using RT-LAMP method from the viewpoint of accurately detecting TYR mRNA in a shorter time with high efficiency.

When RT-LAMP method is used in the detection method of Embodiment 1, TYR mRNA in the sample can be detected by mixing the primer set described above, RNA-dependent DNA polymerase (hereinafter referred to as "reverse transcriptase"), DNA-dependent DNA polymerase having strand displacement activity (hereinafter referred to as "strand displacement DNA polymerase"), dNTPs (including dATP, dGTP, dTTP and dCTP) and a sample, to react them. Therefore, in this case, the above steps (I-1) and (II-1) are carried out in one step.

On the other hand, when RT-LAMP method is used in the detection method of Embodiment 2, TYR mRNA in the sample can be detected by mixing the primer set described above, an enzyme having an action of synthesizing DNA with RNA as a template and an action of synthesizing DNA with DNA as a template while performing strand displacement, dNTPs and a sample, to react them.

As the reverse transcriptase and the strand displacement DNA polymerase, known enzymes can be used.

Furthermore, in the steps (I-1) and (II-1) of the detection method of Embodiment 1 and the step (I-2) of the detection method of Embodiment 2, it is preferred that a buffer providing suitable conditions to an enzyme reaction in each step is also used.

As a sample subjected to TYR mRNA detection, a tissue collected from a living body (lymph node, lymph, blood, and the like), feces, and the like are exemplified.

When RT-LAMP method is used in the detection method of Embodiment 1, the detection of TYR mRNA can be carried out through amplification steps [the above steps (I-1) and (II-1)] and a detection step [the above steps (III-1)].

First, a TYR mRNA-containing sample collected from a living body, the primer set described above, a reverse transcriptase, dNTPs and a strand displacement DNA polymerase are mixed. Moreover, the resulting mixture is heated to a certain temperature (e.g., 65°C), and RT reaction and LAMP reaction are carried out to synthesize TYR cDNA with TYR mRNA as a template and also to additionally synthesize and amplify the TYR cDNA using this TYR cDNA as a template [the above steps (I-1) and (II-1)].

The mechanisms of the RT reaction and the LAMP reaction are as follows.
First, a second sequence of RIP hybridizes with the region R2c of TYR mRNA in a reaction solution. Thereafter, TYR cDNA is synthesized from the 3' end of RIP by the reverse transcriptase with TYR mRNA as a template. Whereby a double-stranded nucleic acid comprising TYR cDNA extended from RIP (RIP extended chain) and TYR mRNA is synthesized.

Next, R3 primer hybridizes with the region R3c of TYR mRNA. Thereafter, TYR cDNA is synthesized from the 3' end of R3 primer by the strand displacement DNA polymerase with TYR mRNA as a template while the RIP extended chain already bound to TYR mRNA is peeled off (while substituted).

Since the RIP extended chain is single-stranded and has a sequence complementary to TYR mRNA, the RIP extended chain comprises the region F2c which is complementary to the region F2 of TYR mRNA. A fourth sequence of FIP hybridizes with this region F2c of the RIP extended chain.

Thereafter, DNA synthesis is carried out from the 3' end of FIP by the strand displacement DNA polymerase with the RIP extended chain as a template. Since the synthesized DNA has the third sequence (polynucleotide sequence that hybridizes with the region F1) at the 5' end, a polynucleotide comprising the third sequence hybridizes with the region F1 on this DNA to form a stem-loop structure. In addition, since this DNA has a sequence complementary to the first sequence (sequence that hybridizes with the region R1c) at the 3' end, the sequence complementary to the first sequence hybridizes with the region R1c on this DNA to form a stem-loop structure. Therefore, this DNA becomes to form a dumbbell structure with the stem-loop structures formed at both 5' end and 3' end.

The DNA with a dumbbell structure (dumbbell DNA) is extended by the strand displacement DNA polymerase from the 3' end with other parts in the dumbbell DNA as a template while the stem-loop structure at the 5' end side is dissociated, to give an extended chain. Since this extended chain has a sequence complementary each other at the 5' end, the 3' end, and the center of these ends, three stem-loop structures are formed in the extended chain. Furthermore, the extended chain is extended by the strand displacement DNA polymerase while the stem-loop structure is dissociated from the 3' end with other parts in the extended chain as a template. This reaction is repeated substantially under isothermal conditions, thereby synthesizing a nucleic acid having plural specific sequences in the molecule (see US Patent No. 6,410,278 and US Patent No. 6,974,670 for the detail of the reaction).

Most of the nucleic acids in the amplification products thus obtained are dsDNA (double-stranded DNA). Therefore, in the detection step [the above step (III-1)], this amplification product can be detected by fluorescently-staining the amplification product with a fluorescent intercalator, for example, ethidium bromide, SYBR (registered trademark) GREEN I, Pico Green (registered trademark) or the like and irradiating with ultraviolet to the product, thereby eliciting fluorescence.

Fluorescent stain may be carried out by adding a fluorescent dye to the reaction solution after amplification reaction [after the above step (II-1)]. In addition, fluorescent stain or may be carried out by previously adding a fluorescent dye to the reaction solution and carrying out nucleic acid amplification in the presence of the fluorescent dye. In the detection step [the above step (III-1)], whether or not TYR mRNA is present in a sample is determined depending on whether or not the fluorescence is detected. Also, quantification of the amplification product can be carried out by measuring the fluorescence intensity of the reaction solution after amplification reaction [after the above step (II-1)]. Based on this quantification result, TYR mRNA contained in a sample can be quantified. In particular, when nucleic acid amplification is carried out in the presence of fluorescent dye, an increase of the fluorescence intensity of the reaction solution is measured in real time, and the time to reach a certain fluorescence intensity is measured, whereby it can be converted to the amount of TYR mRNA in the sample (real-time RT-PCR method, real-time RT-LAMP method, and the like).

In addition, in LAMP reaction in the above step (II-1), magnesium pyrophosphate which is insoluble to water is generated as a byproduct. The magnesium pyrophosphate is increased as nucleic acid amplification proceeds. The reaction solution becomes clouded with an increase of the magnesium pyrophosphate. Therefore, a target nucleic acid can be detected by i) visually confirming the turbidity of the reaction solution, ii) measuring the absorbance or scattered light intensity of the reaction solution to determine the turbidity, or iii) filtering the reaction solution through a colored filter and confirming the residue on the filter (see WO01/83817). When the turbidity is determined by measuring the absorbance or scattered light intensity of the reaction solution, as like the case using the fluorescent dye, the increase in the amplification of DNA and the turbidity can be chased in a closed system by monitoring a change in the turbidity in real time. Therefore, in the detection step [the above step (III-1)], whether or not TYR mRNA is present in a sample can be determined depending on whether or not the reaction solution becomes clouded. Also, quantification of an amplification product can be carried out by determining the turbidity of the amplification product. Based on the quantification result, TYR mRNA contained in a sample can be quantified. When an increase in the turbidity is measured in real time, the time to reach a certain turbidity (hereinafter referred to as amplification rise time) can also be measured to convert the time to the amount of TYR mRNA in the sample.

On the other hand, in the detection method of Embodiment 2, the step (1-2) is an amplification step corresponding to the amplification steps in the detection method of Embodiment 1 [the above steps (I-1) and (II-1)]. This step (1-2) can be carried out in the same manner as in the above steps (I-1) and (II-1) except using one kind of enzyme having an action of synthesizing DNA with RNA as a template and an action of synthesizing DNA with DNA as a template while performing strand displacement, in place of two kinds of enzymes (reverse transcriptase and strand displacement DNA polymerase) in the above steps (I-1) and (II-1).

In addition, in the detection method of Embodiment 2, the step (II-2) can be carried out in the same manner as in the detection step in the detection method of Embodiment 1 [the above step (III-1)].

In the present invention, reagents used in the step (I-1), step (II-1) and step (1-2) described above can be provided as a detection reagent kit for mRNA. This detection reagent kit for mRNA comprises the primer set described above, dNTPs, and an enzyme having an action of synthesizing DNA with RNA as a template and an action of synthesizing DNA with DNA as a template while performing strand displacement or both of RNA-dependent DNA polymerase and DNA-dependent DNA polymerase.

Each reagent described above may be stored in a separate container. In addition, the reverse transcriptase and the strand displacement DNA polymerase may be stored in the same container. Also, at least two kinds of reagents out of dNTPs, buffers, and various primers may be stored in the same container.

Hereinafter, the present invention will be described in detail with reference to Examples, but the present invention is not limited to such Examples.

### (Example 1)

Using the primer sets 1 to 45 as shown in Table 1, whether or not TYR mRNA could be detected was examined as follows.

### (1) Sample Preparation

In a stepwise manner, 5 x 10¹⁰ copies/µL TYR mRNA was diluted with RNase free ultrapure water, to prepare 2.5 x 10³ copies/µL TYR mRNA sample.

### (2) Preparation of Reaction Solution

F3 primer, FIP, RIP and R3 primer as shown in Table 1 were added to a buffer solution [10 mM Tris HCl (pH 8.0)] so as to have the following composition, to prepare a primer mix.
16 µM FIP
16 µM RIP
1 µM F3 primer
1 µM R3 primer
12 µM Loop primer F
12 µM Loop primer R
10 mM Tris HCl (pH 8.0)

In addition, each reagent was mixed so as to have the following composition, to prepare a buffer solution for RT-LAMP reaction.
53 mM Tris HCl (pH 8.8)
1.8 x Thermopol buffer
(manufactured by New England Biolabs, Inc.)
1.43 mM dNTPs
(manufactured by Invitrogen)
5.36 mM MgSO₄
(manufactured by NACALAI TESQUE, INC.)
8.93 mM DTT
(manufactured by Wako Pure Chemical Industries, Ltd.)
2% by volume Tergitol
(manufactured by Sigma)

Next, each reagent was mixed so as to have the following composition, to prepare an enzyme solution (3.04 µL).

| | |
|---|---|
| 10 U/µL AMV reverse transcriptase (manufactured by Promega Corporation) | 0.14 µL |
| 8 U/µL BstDNA polymerase (manufactured by New England Biolabs, Inc.) | 2.27 µL |
| 40 U/µL RNase inhibitor (manufactured by Promega Corporation) | 0.63 µL |

Each reagent was mixed so as to have the following composition, to prepare 25 µL of a reaction solution. The reaction solution was prepared for each of 45 kinds of the primer sets.

| | |
|---|---|
| Buffer solution for RT-LAMP reaction | 14.0 µL |
| Enzyme solution | 3.0 µL |
| Primer mix | 5.0 µL |
| 10 mM Tris HCl (pH 8.0) | 1.0 µL |
| RNA sample | 2.0 µL |

As negative controls (NC) corresponding to each of 45 kinds of the reaction solutions, reaction solutions for NC (45 kinds) to which 2.0 µL of ultrapure water was added in place of 2.0 µL of RNA sample were also prepared.

### (3) Detection and Detection Result

A tube storing a reaction solution containing one of 45 kinds of the primer sets was set to a real time turbidimeter (trade name: LA-200, manufactured by Teramecs Co., Ltd.) previously heated to 65°C. Thereafter, the reaction solution in the tube was incubated at 65°C, to carry out LAMP reaction. The time that had elapsed from the start of reaction until the turbidity of the reaction solution reached 0.1 was determined. The determination of the negative control was once carried out. In addition, the determination of the reaction solution containing the RNA sample was carried out for 3 times (n=3). The determination result of the negative control and the average values of the determination results of the reaction solutions each containing the RNA sample are shown in Table 2. In the table, "NC" shows the determination result of the negative control. Also, in the table, "- 'hyphen'" shows that the turbidity did not reach 0.1 within 60 minutes.

**[Table 2]**

| Primer Set | NC (min) | Determination Result (min) | | Primer Set | NC (min) | Determination Result (min) |
|---|---|---|---|---|---|---|
| 1 | - | 10.33 | | 24 | - | 13.60 |
| 2 | - | 10.57 | | 25 | - | 13.60 |
| 3 | - | 10.63 | | 26 | - | 14.13 |
| 4 | - | 10.70 | | 27 | - | 14.20 |
| 5 | - | 10.83 | | 28 | - | 14.57 |
| 6 | - | 10.97 | | 29 | - | 14.77 |
| 7 | - | 11.00 | | 30 | - | 14.77 |
| 8 | - | 11.03 | | 31 | - | 15.47 |
| 9 | - | 11.03 | | 32 | - | 15.53 |
| 10 | - | 11.17 | | 33 | - | 15.57 |
| 11 | - | 11.17 | | 34 | - | 15.87 |
| 12 | - | 11.30 | | 35 | - | 15.87 |
| 13 | - | 11.37 | | 36 | - | 16.07 |
| 14 | - | 11.37 | | 37 | - | 16.30 |
| 15 | - | 11.50 | | 38 | - | 16.33 |
| 16 | - | 11.53 | | 39 | - | 16.50 |
| 17 | - | 11.60 | | 40 | - | 17.10 |
| 18 | - | 11.67 | | 41 | - | 17.10 |
| 19 | - | 11.83 | | 42 | - | 17.77 |
| 20 | - | 12.17 | | 43 | - | 19.63 |
| 21 | - | 12.20 | | 44 | - | 24.80 |
| 22 | | 12.73 | | 45 | | 30.40 |
| 23 | | 13.43 | | | | |

From the results shown in Table 2, it can been seen that, in the case of the negative controls, nonspecific nucleic acid amplification does not occur in 60 minutes, so that the presence of TYR mRNA can not be detected. However, in the case of the reaction solutions containing the RNA sample, it can be seen that TYR mRNA can be rapidly detected, since the turbidity of the reaction solutions reached 0.1 within 60 minutes, even in a case where any of primer sets 1 to 45 in one embodiment of the present invention was used.

## Claims

1. A primer for detecting tyrosinase mRNA used for detection of tyrosinase mRNA in a sample, comprising a first sequence at the 5' end side and a second sequence at the 3' end side,
wherein the first sequence is defined by the following (a1) and (b1):
(a1) having 10 to 30 nucleotides in length, and
(b1) being a sequence of a polynucleotide capable of hybridizing with a strand complementary to a first region which is a partial region of SEQ ID NO: 1,
the second sequence is defined by the following (c1) and (d1):
(c1) having 10 to 30 nucleotides in length, and
(d1) being a sequence of a polynucleotide capable of hybridizing with a second region located in the 3' end side from the first region in SEQ ID NO: 1, and
the second region is a region comprising:
(i) the 901st and 902nd nucleotides of SEQ ID NO: 1; or
(ii) the 1118th and 1119th nucleotides of SEQ ID NO: 1.

2. The primer according to claim 1, comprising:
(A1) a polynucleotide comprising a nucleotide sequence as shown in any one of SEQ ID NOs: 14 to 19; or
(B1) a polynucleotide having a primer function in a nucleic acid amplification reaction, and having a nucleotide sequence in which one or plural nucleotides are substituted, deleted, inserted or added in the polynucleotide of the item (A1).

3. The primer according to claim 1, which hybridizes with any one of:
a region from the 886th to 908th in SEQ ID NO: 1; i
a region from the 1110th to 1133rd in SEQ ID NO: 1; i
a region from the 1114th to 1131st in SEQ ID NO: 1; and
a region from the 1114th to 1133rd in SEQ ID NO: 1.

4. The primer according to claim 1, wherein the second sequence comprises a sequence consisting of 3 consecutive nucleotides and being completely complementary to a sequence of the second region at the 3' end.

5. A primer set for detecting tyrosinase mRNA used for detecting tyrosinase mRNA in a sample, comprising a first primer, a second primer, and a third primer,
wherein the first primer is the primer of claim 1,
the second primer comprises a third sequence at the 5' end side and a fourth sequence at the 3' end side,
the third sequence is defined by the following (a2) and (b2):
(a2) having 10 to 30 nucleotides in length, and
(b2) being a sequence of a polynucleotide capable of hybridizing with a third region located in the 5' end side from the first region in SEQ ID NO: 1,
the fourth sequence is defined by the following (c2) and (d2):
(c2) having 10 to 30 nucleotides in length, and
(d2) being a sequence of a polynucleotide capable of hybridizing with a strand complementary to a fourth region located in the 5' end side from the third region in SEQ ID NO: 1, and
the third primer is a primer comprising a sequence of a polynucleotide capable of hybridizing with a strand complementary to a fifth region located in the 5' end side from the fourth region in SEQ ID NO: 1.

6. The primer set according to claim 5, wherein the second primer comprises:
(A2) a polynucleotide comprising a nucleotide sequence as shown in any one of SEQ ID NOs: 7 to 13; or
(B2) a polynucleotide having a primer function in a nucleic acid amplification reaction, and having a nucleotide sequence in which one or
plural nucleotides are substituted, deleted, inserted or added in the polynucleotide of the item (A2), and
the third primer comprises:
(A3) a polynucleotide comprising a nucleotide sequence as shown in any one of SEQ ID NOs: 2 to 6; or
(B3) a polynucleotide having a primer function in a nucleic acid amplification reaction, and having a nucleotide sequence in which one or plural nucleotides are substituted, deleted, inserted or added in the polynucleotide of the item (A3).

7. The primer set according to claim 5, wherein the second primer hybridizes with a region complementary to any region of:
a region from the 731st to 751st in SEQ ID NO: 1;
a region from the 963rd to 977th in SEQ ID NO: 1;
a region from the 964th to 986th in SEQ ID NO: 1;
a region from the 971st to 992nd in SEQ ID NO: 1;
a region from the 972nd to 992nd in SEQ ID NO^{:} 1; and
a region from the 986th to 1008th in SEQ ID NO: 1.

8. The primer set according to claim 5, wherein the third primer hybridizes with a region complementary to any region of:
a region from the 703rd to 722nd in SEQ ID NO: 1;
a region from the 931st to 953rd in SEQ ID NO: 1;
a region from the 945th to 962nd in SEQ ID NO: 1;
a region from the 950th to 962nd in SEQ ID NO: 1; and
a region from the 966th to 985th in SEQ ID NO: 1.

9. The primer set according to claim 5, further comprising a fourth primer capable of hybridizing with a sixth region located in the 3' end side from the second region in SEQ ID NO: 1.

10. The primer set according to claim 9, wherein the fourth primer comprises:
(A4) a polynucleotide comprising a nucleotide sequence as shown in any one of SEQ ID NOs: 20 to 25; or
(B4) a polynucleotide having a primer function in a nucleic acid amplification reaction, and having wherein a nucleotide sequence in which one or plural nucleotides are substituted, deleted, inserted or added in the polynucleotide of the item (A4).

11. The primer set according to claim 9, wherein the fourth primer hybridizes with any one of:
a region from the 917th to 936th in SEQ ID NO: 1;
a region from the 1135th to 1153rd in SEQ ID NO: 1;
a region from the 1138th to 1155th in SEQ ID NO: 1;
a region from the 1138th to 1156th in SEQ ID NO: 1;
a region from the 1141st to 1160th in SEQ ID NO: 1; and
a region from the 1142nd to 1161st in SEQ ID NO: 1.

12. The primer set according to claim 5, further comprising a fifth primer that hybridizes with a seventh region located between the third region and the fourth region in SEQ ID NO: 1.

13. The primer set according to claim 12, wherein the fifth primer comprises:
(A5) a polynucleotide as shown in any one of SEQ ID NOs: 26 to 55; or
(B5) a polynucleotide having a primer function in a nucleic acid amplification reaction, having a nucleotide sequence in which one or plural nucleotides have a substituted, deleted, inserted or added in the polynucleotide of the item (A5).

14. The primer set according to claim 12, wherein the fifth primer hybridizes with any one of:
a region from the 752nd to 776th in SEQ ID NO: 1;
a region from the 981st to 1004th in SEQ ID NO: 1;
a region from the 982nd to 1004th in SEQ ID NO: 1;
a region from the 982nd to 1006th in SEQ ID NO: 1;
a region from the 986th to 1010th in SEQ ID NO: 1;
a region from the 987th to 1011st in SEQ ID NO: 1;
a region from the 988th to 1011st in SEQ ID NO: 1;
a region from the 988th to 1012nd in SEQ ID NO: 1;
a region from the 989th to 1011st in SEQ ID NO: 1;
a region from the 989th to 1012nd in SEQ ID NO: 1;
a region from the 989th to 1013rd in SEQ ID NO: 1;
a region from the 990th to 1012nd in SEQ ID NO: 1;
a region from the 990th to 1013rd in SEQ ID NO: 1;
a region from the 990th to 1014th in SEQ ID NO: 1;
a region from the 992nd to 1015th in SEQ ID NO: 1;
a region from the 993rd to 1016th in SEQ ID NO: 1;
a region from the 994th to 1016th in SEQ ID NO: 1;
a region from the 995th to 1016th in SEQ ID NO: 1;
a region from the 995th to 1018th in SEQ ID NO: 1;
a region from the 996th to 1018th in SEQ ID NO: 1;
a region from the 1000th to 1019th in SEQ ID NO: 1;
a region from the 1001st to 1020th in SEQ ID NO: 1;
a region from the 1003rd to 1022nd in SEQ ID NO: 1;
a region from the 1004th to 1022nd in SEQ ID NO: 1;
a region from the 1005th to 1024th in SEQ ID NO: 1;
a region from the 1006th to 1024th in SEQ ID NO: 1;
a region from the 1008th to 1027th in SEQ ID NO: 1;
a region from the 1009th to 1027th in SEQ ID NO: 1;
a region from the 1009th to 1028th in SEQ ID NO: 1; and
a region from the 1010th to 1029th in SEQ ID NO: 1.

15. The primer set according to claim 5, further comprising a sixth primer that hybridizes with a region complementary to an eighth region located between the first region and the second region in SEQ ID NO: 1.

16. The primer set according to claim 15, wherein the sixth primer comprises:
(A6) a polynucleotide as shown in any one of SEQ ID NOs: 56 to 75; or
(B6) a polynucleotide having a primer function in a nucleic acid amplification reaction, having a nucleotide sequence in which one or plural nucleotides are substituted, deleted, inserted or added in the polynucleotide of the item (A6).

17. The primer set according to claim 15, wherein the sixth primer hybridizes with a region complementary to any region of:
a region from the 860th to 884th in SEQ ID NO: 1;
a region from the 1063rd to 1087th in SEQ ID NO: 1;
a region from the 1065th to 1089th in SEQ ID NO: 1;
a region from the 1066th to 1090th in SEQ ID NO: 1;
a region from the 1067th to 1089th in SEQ ID NO: 1;
a region from the 1067th to 1090th in SEQ ID NO: 1;
a region from the 1068th to 1091st in SEQ ID NO: 1;
a region from the 1068th to 1092nd in SEQ ID NO: 1;
a region from the 1069th to 1091st in SEQ ID NO: 1;
a region from the 1069th to 1092nd in SEQ ID NO: 1;
a region from the 1069th to 1093rd in SEQ ID NO: 1;
a region from the 1070th to 1093rd in SEQ ID NO: 1;
a region from the 1070th to 1094th in SEQ ID NO: 1;
a region from the 1073rd to 1097th in SEQ ID NO: 1;
a region from the 1074th to 1098th in SEQ ID NO: 1;
a region from the 1075th to 1099th in SEQ ID NO: 1;
a region from the 1082nd to 1104th in SEQ ID NO: 1;
a region from the 1083rd to 1105th in SEQ ID NO: 1;
a region from the 1084th to 1105th in SEQ ID NO: 1; and
a region from the 1085th to 1107th in SEQ ID NO: 1.

18. A detection reagent kit for tyrosinase mRNA comprising:
the primer set of claim 5,
dNTPs, and
an enzyme having an action of synthesizing DNA with RNA as a template and an action of synthesizing DNA with DNA as a template while performing strand displacement, or both of RNA-dependent DNA polymerase and DNA-dependent DNA polymerase.

19. The detection reagent kit for tyrosinase mRNA according to claim 18, wherein the second primer comprises:
(A2) a polynucleotide comprising a nucleotide sequence as shown in any one of SEQ ID NOs: 7 to 13; or
(B2) a polynucleotide having a primer function in a nucleic acid amplification reaction, and having a nucleotide sequence in which one or
plural nucleotides are substituted, deleted, inserted or added in the polynucleotide of the item (A2), and
the third primer comprises:
(A3) a polynucleotide comprising a nucleotide sequence as shown in any one of SEQ ID NOs: 2 to 6; or
(B3) a polynucleotide having a primer function in a nucleic acid amplification reaction, and having a nucleotide sequence in which one or plural nucleotides are substituted, deleted, inserted or added in the polynucleotide of the item (A3).

20. A method for detecting tyrosinase mRNA, comprising the steps of:
reacting a sample with the primer set of claim 5 and RNA-dependent DNA polymerase, to synthesize cDNA from tyrosinase mRNA in the sample; i
reacting the cDNA synthesized in the above step with the primer set and DNA-dependent DNA polymerase , to amplify the cDNA; and
detecting the cDNA amplified in the above step, thereby detecting tyrosinase mRNA in the sample.

21. A method for detecting tyrosinase mRNA, comprising the steps of:
reacting a sample with the primer set of claim 5 and an enzyme having an action of synthesizing DNA with RNA as a template and an action of synthesizing DNA with DNA as a template while performing strand displacement, to synthesize cDNA with tyrosinase mRNA in the sample as a template and to additionally synthesize and amplify the cDNA with the cDNA as a template; and
detecting the cDNA amplified in the above step, thereby detecting tyrosinase mRNA in the sample.
